# EUROPEAN PATENT SPECIFICATION

(11) **EP 2 775 975 B1**
(45) Date of publication and mention of the grant of the patent: **23.03.2016**
(21) Application number: 12794508.7
(22) Date of filing: 25.10.2012
(51) Int. Cl.: B01J 20/32, A61F 13/15

(54) **APPARATUS AND PROCESS FOR FORMING ABSORBENT CORES FOR ABSORBENT SANITARY PRODUCTS**
GERÄT UND VERFAHREN ZUR BILDUNG VON SAUGFÄHIGEN KERNEN FÜR ABSORBIERENDE HYGIENEARTIKEL
APPAREIL ET PROCÉDÉ POUR LA FORMATION DE NOYAUX ABSORBANTS POUR DES PRODUITS SANITAIRES ABSORBANTS

(30) Priority: 08.11.2011 IT TO20111028
(43) Date of publication of application: 17.09.2014
(73) Proprietor: Fameccanica.Data S.p.A., 65129 Pescara (IT)
(72) Inventor: D'APONTE, Francesco, I-65120 Pescara (IT); DE LAURETIS, Giacomo, I-64014 Martinsicuro (Teramo) (IT)
(74) Representative: Marchitelli, Mauro
(86) International application number: PCT/IB2012/055884
(87) International publication number: WO 2013/068869

(56) References cited:
- US-A- 5 025 910
- US-A1- 2006 081 348

## Description

### TEXT OF THE DESCRIPTION

### Field of the invention

The present invention relates to the production of absorbent sanitary products and regards an apparatus and a process for forming absorbent cores that include cellulose fluff and superabsorbent polymers.

### Description of the relevant art

A conventional technique for forming absorbent cores that include cellulose fluff and superabsorbent polymers consists in forming a continuous moving chain including a succession of pads of cellulose fluff oriented parallel to the direction of movement of the chain. Dispensed amounts of superabsorbent polymers are applied on the moving absorbent pads. Typically, the formation of the absorbent pads of cellulose fluff and the application of the superabsorbent polymers are made on a forming wheel that is able to turn about its own axis at a peripheral speed equal to the speed of advance of the continuous chain.

Conventional techniques for the formation of absorbent pads with superabsorbent polymers envisage a substantially constant concentration of the superabsorbent polymers on the surface of the pads.

It would be desirable to vary the concentration of the superabsorbent polymers in the longitudinal direction of the absorbent cores, for example to have a high concentration of superabsorbent polymers in the central area of the core and a low or zero concentration of superabsorbent polymers at the longitudinal ends of the absorbent cores. In this way, there would be a greater concentration of superabsorbent polymers in the areas in which during use there is a greater concentration of liquids.

With the techniques for forming absorbent cores of a conventional type, where the pads of cellulose fluff are oriented parallel to the direction of movement, it is very difficult to vary the concentration of the superabsorbent polymers in the longitudinal direction of the pads. In fact, to vary the concentration of superabsorbent polymers in the longitudinal direction on pads that move parallel to their longitudinal direction it would be necessary to provide dispensers capable of varying the amount of superabsorbent polymers supplied as a function of time, which would involve a considerable constructional complexity. Moreover, the precision of the distribution of concentration of the superabsorbent polymers would be approximate, especially on high-speed lines.

### Object and summary of the invention

The object of the present invention is to provide an apparatus and a process that will enable a distribution of superabsorbent polymers to be obtained with a non-uniform concentration in the longitudinal direction of the absorbent cores, with a structure that is simple and ensures a high precision of the law of distribution, even on high-speed lines.

According to the present invention, said object is achieved by an apparatus and by a process having the characteristics that form the subject of Claims 1 and 7.

### Brief description of the drawings

The present invention will now be described in detail with reference to the attached drawings, which are provided purely by way of non-limiting example and in which:
- Figure 1 is a schematic side view of an apparatus for forming absorbent cores according to the present invention;
- Figure 2 is a schematic top plan view illustrating the sequence of the steps of the process of formation of absorbent cores according to the invention;
- Figure 3 is a schematic cross-sectional view according to the line III-III of Figure 1; and
- Figure 4 is an enlarged detail of the part indicated by the arrow IV in Figure 3.

### Description of preferred embodiments

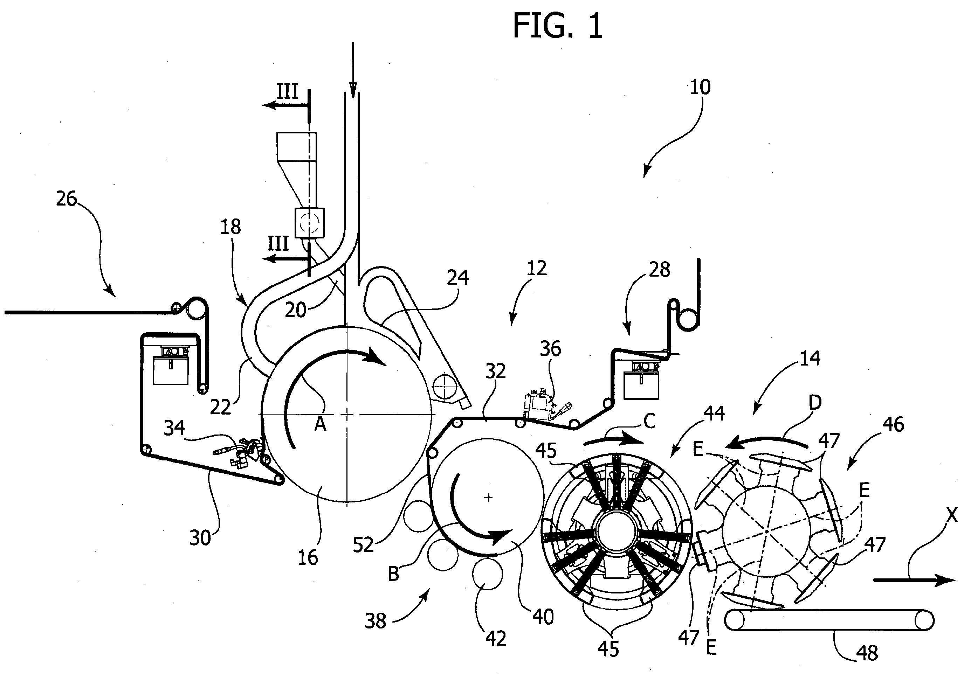

With reference to Figure 1, designated by 10 is an apparatus for the production of absorbent cores, forming part of a line for production of absorbent sanitary products. The apparatus 10 comprises a forming section 12 in which the absorbent cores are formed, and an application station 14 in which the absorbent cores coming from the section 12 are applied to a continuous supporting web moving in the longitudinal direction X. The forming section 12 comprises a forming wheel 16, which is able to turn about a transverse axis orthogonal to the longitudinal direction X in the direction indicated by the arrow A. The forming wheel 16 is provided on its periphery with holes connected to a suction source.

The forming section 12 comprises a first duct 18 for feeding cellulose fluff to the forming wheel 16 and a second duct 20 for feeding dispensed amounts of superabsorbent polymers. The first duct 18 has one end 22 facing the periphery of the forming wheel 16. The second duct 20 comprises a distribution chamber 24 facing a portion of the periphery of the forming wheel 16 situated downstream with respect to the end 22 of the duct 18.

Preferably, the forming section 12 comprises two web-feeding assemblies 26, 28, configured for supplying, respectively, a first web 30 and a second web 32 at the periphery of the forming wheel 16. The first web 30 is fed at the periphery of the forming wheel 16 upstream of the end 22 of the duct 18. The second web 32 is fed at the periphery of the forming wheel 16 downstream of the distribution chamber 24. Preferably, a first dispenser 34 and a second dispenser 36 are arranged for applying respective layers of glue on the first web 30 and on the second web 32, respectively. At least one of the webs 30, 32 is made of permeable material, for example non-woven fabric or tissue paper.

The forming section 12 comprises a cutting unit 38 including a transfer wheel 40 and a cutting roller 42 co-operating with the outer surface of the transfer wheel 40. The transfer wheel 40 turns in the direction indicated by the arrow B at a peripheral speed V1 equal to the peripheral speed of the forming wheel 16.

The application station 14 comprises a phasing unit 44 and a rotation unit 46 that co-operates with a belt conveyor 48.

The phasing unit comprises a plurality of gripping elements 45, that are able to turn about a common transverse axis in the direction indicated by the arrow C. Each of the gripping elements over one complete revolution accelerates cyclically from a peripheral speed V1 to a peripheral speed V2 and then decelerates from the peripheral speed V2 to the peripheral speed V1.

The rotation unit 46 comprises a plurality of radial arms 47 that turn jointly at a peripheral speed V2 in the direction indicated by the arrow D. Each of the arms 47 has a gripping element that is able to turn through 90° about a respective radial axis E between a position of gripping and a position of release.

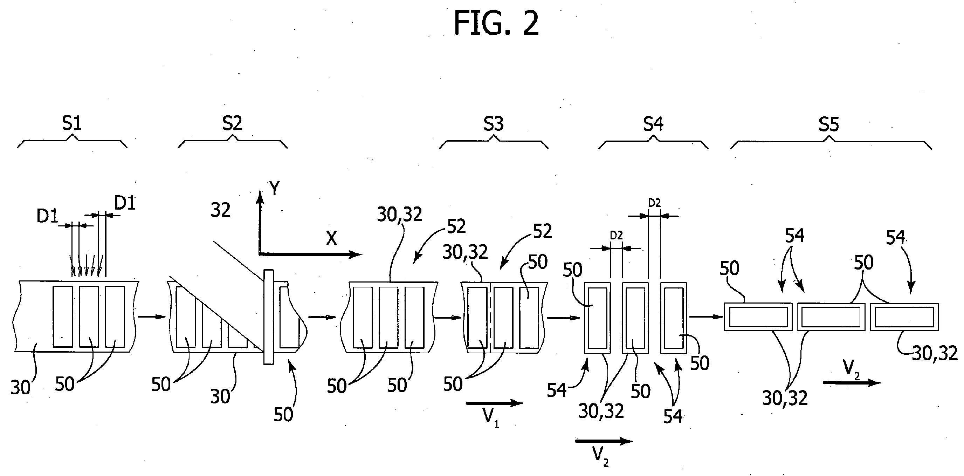

Operation of the apparatus 10 is schematically represented in Figure 2.

In a first step S1, the continuous web 30 is fed at the periphery of the forming wheel 16. Applied on the web 30 are a series of absorbent pads 50 of cellulose fluff. The absorbent pads 50 extend in length in a direction Y orthogonal with respect to the direction X in which the web 30 advances. The pads of cellulose fluff 50 are formed thanks to the suction through the peripheral surface of the forming wheel 16. The layers of cellulose fluff 50 remain connected to the web 30 thanks to the layer of glue present on the top surface of the web 30. The absorbent pads of cellulose fluff 50 are set apart from one another in the direction X by a distance D1. The spacing between the pads 50 can be obtained, for example envisaging areas without holes on the outer surface of the forming wheel 16.

In a second step S2, the second web 32 is applied on the first web 30. The two webs 30, 32 are set side by side and pressed together and remain glued together around the absorbent pads 50. At output from the forming wheel 16, there is hence obtained a continuous chain 52 formed by a continuous succession of absorbent pads 50 enclosed between the two webs 30, 32 and oriented in a direction transverse with respect to the direction of movement X of the chain 52. The continuous chain 52 advances in the direction X at a first speed V1.

In a third step S3, in the cutting unit 38 the continuous chain 52 is cut crosswise in the spaces between the absorbent pads 50 so as to form individual absorbent cores 54 separate from one another, comprising respective absorbent pads 50 enclosed between two webs 30, 32 joined together along the perimeter of the absorbent pads 50.

In a fourth step S4, downstream of the cutting unit 38 the phasing unit 44 picks up the individual absorbent cores 54 separated from the continuous chain 52 at the speed V1 and accelerates them to a speed V2 greater than the speed V1. The effect of the acceleration from the speed V1 to the speed V2 is that the individual absorbent cores 54 are set apart from one another in the direction X by a distance D2 greater than the distance D1 between the absorbent pads 50 in the continuous chain 52. In the phasing unit 44 the individual absorbent cores 54 are still oriented in a direction transverse with respect to the direction of advance X.

In a fifth step S5, the rotation unit 46 picks up the individual absorbent cores 54 oriented in a direction transverse to the direction of advance and turns them through 90°, arranging them parallel to the direction of advance X.

The rotation unit 46 turns in phase with the phasing unit 44 and picks up from the latter the absorbent cores 54 at the speed V2. After rotation of the absorbent cores 54 through 90°, the rotation unit 46 applies the cores 54 oriented parallel to the direction X on a continuous supporting web moving on the conveyor 48 in the direction X at the speed V2.

Application of superabsorbent polymers to the absorbent pads 50 of cellulose fluff is made with a non-constant concentration in the direction Y transverse with respect to the direction X of advance of the continuous chain 52. The concentration of superabsorbent polymers can be expressed in milligrams or cubic millimetres of superabsorbent product per unit surface (square millimetres or square centimetres) of the absorbent pad 50. The fact that the superabsorbent polymers are applied to the absorbent pads 50 whilst these are oriented in a direction transverse to the direction of their movement enables in a simple way variation of the concentration of the superabsorbent polymers in a transverse direction.

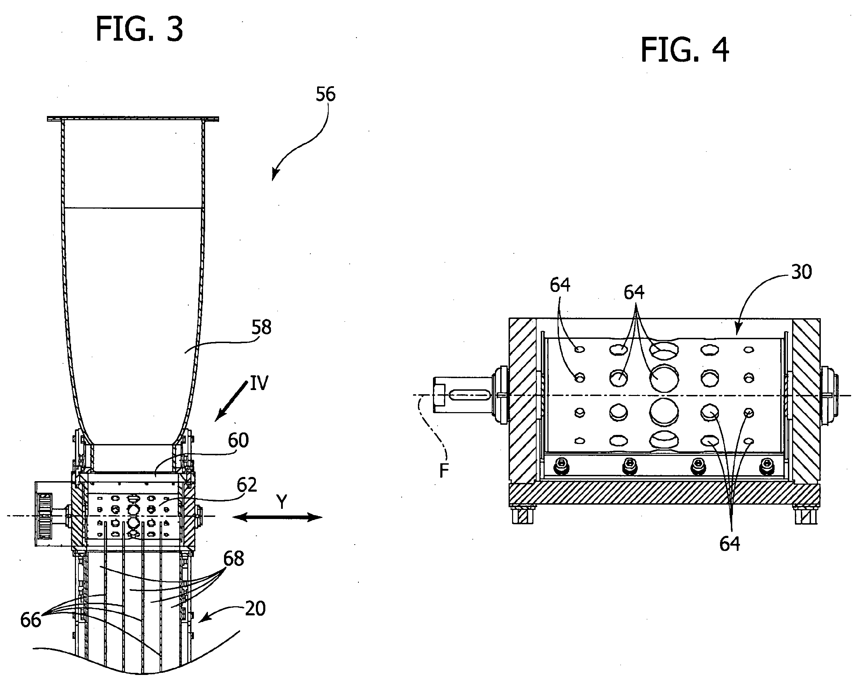

Illustrated in Figures 3 and 4 is an example of the way in which it is possible to apply the superabsorbent polymers with variable concentration in a transverse direction. With reference to Figure 3, designated by 56 is a device for dispensing superabsorbent polymers. The dispenser device 56 is connected to the top end of the duct 20. The dispenser device 56 comprises a container 58 in which the superabsorbent polymers are contained, generally in the form of powders. The container 58 is open at the bottom and communicates with a chamber 60 in which a dispenser drum 62 is housed, which is able to turn about an axis F parallel to the transverse direction Y. The dispenser drum 62 is provided, on its outer surface, with holes with blind bottoms 64, illustrated in greater detail in Figure 4. The holes 64 are grouped in circumferential arrays set apart from one another in a transverse direction. The holes of the various arrays have diameters different from one another.

With reference to Figure 3, the channel 20 for feeding superabsorbent polymers is provided, inside, with partitions 66 that divide the channel 20 into a series of passages 68 set apart from one another in the transverse direction Y. The various passages 68 communicate with respective arrays of holes 64.

In operation, the dispenser drum 62 is driven in rotation about its own axis F, with a speed-controlled motor. The holes 64 take superabsorbent polymers out of the container 58 and transfer the polymers contained in the holes 64 into the respective passages 68. The various passages 68 receive different amounts of superabsorbent polymers. The passages 68 communicate with respective portions of the outer surface of the forming wheel 16. In this way, applied to the pads 50 of cellulose fluff that move on the periphery of the forming wheel 16 are amounts of superabsorbent polymers per unit surface that vary in a transverse direction Y.

The dispenser device 56 represents just one example amongst the many possible ones of applying superabsorbent polymers with different concentration in a transverse direction. An alternative way could envisage a series of distributing wheels set alongside one another in a transverse direction and turning in a concordant way or at different speeds about a transverse axis. As further alternative, the various passages 68 could be associated to respective nozzles that feed different flowrates of superabsorbent polymers.

Of course, without prejudice to the principle of the invention, the details of construction and the embodiments may vary widely with respect to what has been described and illustrated herein, without thereby departing from the scope of the invention as defined in the ensuing claims.

## Claims

1. An apparatus for forming absorbent cores (54) that include absorbent pads (50) of cellulose fluff and superabsorbent polymers, comprising a forming section (12) configured for forming a continuous moving chain (52) including a continuous succession of absorbent pads (50) oriented in a direction transverse with respect to the direction of movement (X) of the chain (52), the forming section (12) comprising a dispenser device (56) configured for applying superabsorbent polymers on said absorbent pads (50) oriented in a transverse direction at a variable concentration in a direction (Y) transverse with respect to said direction of movement (X) of the continuous chain (52).

2. The apparatus according to Claim 1, wherein said forming section (12) comprises a forming wheel (16) that is able to turn about a transverse axis, a first duct (18) for feeding cellulose fluff at the periphery of said forming wheel (16) and a second duct (20) for feeding superabsorbent polymers at the periphery of said forming wheel (16) downstream of said first duct (18), the second duct (20) being divided into passages (68) aligned transversely with respect to one another, which in use receive different flowrates of superabsorbent polymers.

3. The apparatus according to Claim 2, wherein said forming section (12) comprises a first web-feeding assembly (26) and a second web-feeding assembly (28), which are configured for supplying a first web (30) and a second web (32) at the periphery of said forming wheel (16) upstream of said first duct (18) and downstream of said second duct (20), respectively.

4. The apparatus according to any one of the preceding claims, wherein the forming section (12) comprises a cutting unit (38) configured for cutting said continuous chain (52) crosswise and for separating individual absorbent cores (54) from said continuous chain (52).

5. The apparatus according to Claim 4, further comprising an application station (14) including a phasing unit (44) configured for picking up individual absorbent cores (54) at output from said cutting unit (38) at a first speed (V1) and for accelerating said absorbent cores (54) to a second speed (V2).

6. The apparatus according to Claim 5, wherein said application station (14) comprises a rotation unit (46) configured for rotating through 90° said absorbent cores (54) and for orienting them parallel to said direction of movement (X).

7. A process for forming absorbent cores (54) that include cellulose fluff and superabsorbent polymers, comprising the steps of:
- forming a continuous moving chain (52) including a succession of absorbent pads (50) of cellulose fluff oriented in a direction transverse to the direction of movement (X) of said continuous chain (52); and
- applying superabsorbent polymers on said absorbent pads (50) of cellulose fluff oriented in a transverse direction at a variable concentration in a transverse direction.

8. The process according to Claim 7, comprising the steps of: feeding a first continuous web (30), applying said absorbent pads (50) of cellulose fluff (50) on said first continuous web (30), applying superabsorbent polymers on said absorbent pads of cellulose fluff with a variable concentration in a transverse direction, applying a second continuous web (32) on said first web (30), and joining together said first and second webs (30, 32) around said absorbent pads (50).

9. The process according to Claim 8, comprising the steps of cutting said continuous moving chain (52) crosswise so as to separate individual absorbent cores (54) from said chain (52), accelerating said individual absorbent cores (54) from a first speed (V1) to a second speed (V2), and rotating said individual absorbent cores (54) through 90° so as to orient said individual absorbent cores (54) parallel to said direction of movement (X).

10. The process according to any one of Claims 7 to 9, wherein said step of application of superabsorbent polymers comprises application of different flowrates of superabsorbent polymers in a series of passages (68) set alongside one another in a transverse direction (Y).

## Patentansprüche

1. Gerät zur Bildung von saugfähigen Kernen (54), die saugfähige Polster (50) aus Zelluloseflaum und hochsaugfähige Polymere umfassen, umfassend: einen Bildungsabschnitt (12), der zum Bilden einer ununterbrochenen sich bewegenden Kette (52) ausgestaltet ist, die eine ununterbrochene Folge saugfähiger Polster (50) umfasst, die in einer Richtung quer zur Bewegungsrichtung (X) der Kette (52) ausgerichtet sind, wobei der Bildungsabschnitt (12) eine Abgabevorrichtung (56) umfasst, die zum Aufbringen hochsaugfähiger Polymere auf die in einer Querrichtung ausgerichteten saugfähigen Polster (50) mit einer variablen Konzentration in einer Richtung (Y) quer zur Bewegungsrichtung (X) der ununterbrochenen Kette (52) ausgestaltet ist.

2. Gerät nach Anspruch 1, wobei der Bildungsabschnitt (12) ein Bildungsrad (16), das in der Lage ist, sich um eine Querachse zu drehen, einen ersten Kanal (18) zum Zuführen von Zelluloseflaum an der Peripherie des Bildungsrades (16) und einen zweiten Kanal (20) zum Zuführen hochsaugfähiger Polymere an der Peripherie des Bildungsrades (16) unterhalb des ersten Kanals (18) umfasst, wobei der zweite Kanal (20) in quer zueinander ausgerichtete Durchlässe (68) unterteilt ist, die bei Verwendung verschiedene Volumenströme hochsaugfähiger Polymere aufnehmen.

3. Gerät nach Anspruch 2, wobei der Bildungsabschnitt (12) eine erste Vlieszuführanordnung (26) und eine zweite Vlieszuführanordnung (28) umfasst, die zum Zuführen eines ersten Vlieses (30) und eines zweiten Vlieses (32) an der Peripherie des Bildungsrades (16) oberhalb des ersten Kanals (18) bzw. unterhalb des zweiten Kanals (20) ausgestaltet sind.

4. Gerät nach einem der vorangehenden Ansprüche, wobei der Bildungsabschnitt (12) eine Schneideinheit (38) umfasst, die zum Schneiden der ununterbrochenen Kette (52) in Querrichtung und zum Abtrennen einzelner saugfähiger Kerne (54) von der ununterbrochenen Kette (52) ausgestaltet ist.

5. Gerät nach Anspruch 4, das weiterhin eine Aufbringstation (14) umfasst, die eine Phasenabgleichseinheit (44) umfasst, die zum Aufnehmen einzelner saugfähiger Kerne (54) am Ausgang aus der Schneideinheit (38) mit einer ersten Geschwindigkeit (V1) und zum Beschleunigen der saugfähigen Kerne (54) auf eine zweite Geschwindigkeit (V2) ausgestaltet ist.

6. Gerät nach Anspruch 5, wobei die Aufbringstation (14) eine Dreheinheit (46) umfasst, die zum Drehen der saugfähigen Kerne (54) um 90° und zum Ausrichten derselben parallel zur Bewegungsrichtung (X) ausgestaltet ist.

7. Verfahren zur Bildung saugfähiger Kerne (54), die Zelluloseflaum und hochsaugfähige Polymere umfassen, das folgende Schritte umfasst:
- Bilden einer ununterbrochenen sich bewegenden Kette (52), die eine Folge saugfähiger Polster (50) aus Zelluloseflaum umfasst, die in einer Richtung quer zur Bewegungsrichtung (X) der ununterbrochenen Kette (52) ausgerichtet sind; und
- Aufbringen hochsaugfähiger Polymere auf die in einer Querrichtung ausgerichteten saugfähigen Polster (50) aus Zelluloseflaum mit einer variablen Konzentration in einer Querrichtung.

8. Verfahren nach Anspruch 7, das folgende Schritte umfasst: Zuführen eines ersten Endlosvlieses (30), Aufbringen der saugfähigen Polster (50) aus Zelluloseflaum (50) auf das erste Endlosvlies (30), Aufbringen hochsaugfähiger Polymere auf die saugfähigen Polster aus Zelluloseflaum mit einer variablen Konzentration in einer Querrichtung, Aufbringen eines zweiten Endlosvlieses (32) auf das erste Vlies (30) und Verbinden des ersten und zweiten Vlieses (30, 32) um die saugfähigen Polster (50) herum.

9. Verfahren nach Anspruch 8, das die Schritte des Schneidens der ununterbrochenen sich bewegenden Kette (52) in Querrichtung, um einzelne saugfähige Kerne (54) von der Kette (52) abzutrennen, Beschleunigens der einzelnen saugfähigen Kerne (54) von einer ersten Geschwindigkeit (V1) auf eine zweite Geschwindigkeit (V2) und Drehens der einzelnen saugfähigen Kerne (54) um 90°, um die einzelnen saugfähigen Kerne (54) parallel zur Bewegungsrichtung (X) auszurichten, umfasst.

10. Verfahren nach einem der Ansprüche 7 bis 9, wobei der Schritt der Aufbringung hochsaugfähiger Polymere eine Aufbringung verschiedener Volumenströme hochsaugfähiger Polymere in einer Reihe von Durchlässen (68), die nebeneinander in einer Querrichtung (Y) angeordnet sind, umfasst.

## Revendications

1. Appareil destiné à la formation de noyaux absorbants (54) qui comprennent des tampons absorbants (50) de duvet de cellulose et de polymères superabsorbants, comprenant une partie de formation (12) configurée pour former une chaîne mobile continue (52) comprenant une succession continue de tampons absorbants (50) orientés dans une direction transversale par rapport à la direction de mouvement (X) de la chaîne (52), la partie de formation (12) comprenant un dispositif distributeur (56) configuré pour appliquer des polymères superabsorbants sur lesdits tampons absorbants (50) orientés dans une direction transversale à une concentration variable dans une direction (Y) transversale par rapport à ladite direction de mouvement (X) de la chaîne continue (52).

2. Appareil selon la revendication 1, dans lequel ladite partie de formation (12) comprend une roue de formation (16) qui est capable de tourner autour d'un axe transversal, un premier conduit (18) permettant d'apporter le duvet de cellulose à la périphérie de ladite roue de formation (16) et un second conduit (20) permettant d'apporter les polymères superabsorbants à la périphérie de ladite roue de formation (16) en aval dudit premier conduit (18), le second conduit (20) étant divisé en passages (68) alignés transversalement les uns par rapport aux autres, qui lors de l'utilisation reçoivent différents débits de polymères superabsorbants.

3. Appareil selon la revendication 2, dans lequel ladite partie de formation (12) comprend un premier ensemble d'alimentation de bande (26) et un second ensemble d'alimentation de bande (28), qui sont configurés pour apporter une première bande (30) et une seconde bande (32) à la périphérie de ladite roue de formation (16) en amont dudit premier conduit (18) et en aval dudit second conduit (20), respectivement.

4. Appareil selon l'une quelconque des revendications précédentes, dans lequel la partie de formation (12) comprend une unité de coupe (38) configurée pour couper ladite chaîne continue (52) transversalement et pour séparer les noyaux absorbants individuels (54) de ladite chaîne continue (52).

5. Appareil selon la revendication 4, comprenant en outre une station d'application (14) comprenant une unité de phasage (44) configurée pour prélever les noyaux absorbants individuels (54) à la sortie de ladite unité de coupe (38) à une première vitesse (V1) et pour accélérer lesdits noyaux absorbants (54) jusqu'une seconde vitesse (V2).

6. Appareil selon la revendication 5, dans lequel ladite station d'application (14) comprend une unité de rotation (46) configurée pour faire pivoter de 90° lesdits noyaux absorbants (54) et pour les orienter parallèlement à ladite direction de mouvement (X).

7. Procédé destiné à la formation de noyaux absorbants (54) qui comprennent un duvet de cellulose et des polymères superabsorbants, comprenant les étapes suivantes :
- formation d'une chaîne mobile continue (52) comprenant une succession de tampons absorbants (50) de duvet de cellulose orientés dans une direction transversale par rapport à la direction de mouvement (X) de ladite chaîne continue (52) ; et
- application de polymères superabsorbants sur lesdits tampons absorbants (50) de duvet de cellulose orientés dans une direction transversale à une concentration variable dans une direction transversale.

8. Procédé selon la revendication 7, comprenant les étapes suivantes : apport d'une première bande continue (30), application desdits tampons absorbants (50) de duvet de cellulose (50) sur ladite première bande continue (30), application des polymères superabsorbants sur lesdits tampons absorbants de duvet de cellulose avec une concentration variable dans une direction transversale, application d'une seconde bande continue (32) sur ladite première bande (30), et assemblage desdites première et seconde bandes (30, 32) autour desdits tampons absorbants (50).

9. Procédé selon la revendication 8, comprenant les étapes consistant à couper ladite chaîne mobile continue (52) transversalement de manière à séparer les noyaux absorbants individuels (54) de ladite chaîne (52), accélérer lesdits noyaux absorbants individuels (54) d'une première vitesse (V1) à une seconde vitesse (V2), et faire pivoter lesdits noyaux absorbants individuels (54) de 90° de manière à orienter lesdits noyaux absorbants individuels (54) parallèlement à ladite direction de mouvement (X).

10. Procédé selon l'une quelconque des revendications 7 à 9, dans lequel ladite étape d'application de polymères superabsorbants comprend l'application de différents débits de polymères superabsorbants dans une série de passages (68) disposés les uns le long des autres dans une direction transversale (Y).
